# FASCICULE DE BREVET EUROPEEN

(11) **EP 2 424 899 B1**
(45) Date de publication et mention de la délivrance du brevet: **08.05.2019**
(21) Numéro de dépôt: 10727063.9
(22) Date de dépôt: 29.04.2010
(51) Int. Cl.: C12P 19/04, C12P 19/14, B08B 9/08, A61M 1/28, A61L 2/00, C08B 30/18, B01D 61/14, C11D 11/00

(54) **PROCEDE DE PURIFICATION DE POLYMERES DE GLUCOSE DESTINES AUX SOLUTIONS DE DIALYSE PERITONEALE**
VERFAHREN ZUR REINIGUNG VON GLUCOSE-POLYMEREN FÜR PERITONEALE- DIALYSE-LÖSUNGEN
PROCESS FOR THE PURIFICATION OF GLUCOSE POLYMERS FOR PERITONEAL DIALYSIS SOLUTIONS

(30) Priorité: 30.04.2009 FR 0952879
(43) Date de publication de la demande: 07.03.2012
(73) Titulaire: Roquette Frères, 62136 Lestrem (FR)
(72) Inventeur: BIGUET, Marc, F-62840 Neuve Chapelle (FR); BOURDAIN, Stéphane, F-62400 Bethune (FR); DUFLOT, Pierrick, F-62136 La Couture (FR)
(74) Mandataire: Cabinet Plasseraud
(86) Numéro de dépôt international: PCT/FR2010/050815
(87) Numéro de publication internationale: WO 2010/125315

(56) Documents cités:
- EP-A- 0 667 356
- EP-A- 1 006 128
- EP-A- 1 108 434
- EP-A- 1 369 432
- WO-A1-97/02753
- WO-A1-2005/102546
- US-A- 3 928 135
- US-A1- 2004 194 810
- US-A1- 2005 142 167
- US-A1- 2008 105 282
- US-B1- 6 284 140

## Description

La présente invention se rapporte à un procédé de purification de polymères de glucose destinés à la fabrication de solutions de dialyse péritonéale.

La dialyse péritonéale est un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

La dialyse péritonéale utilise deux principes mis en action grâce à la propriété physiologique de perméabilité du péritoine : l'ultrafiltration de liquide et l'épuration des déchets par diffusion.

Le péritoine est une membrane séreuse, d'une surface de 2 m² environ, composée de deux feuillets : le feuillet pariétal tapissant la face interne des parois (abdomen, petit bassin, diaphragme) et le feuillet viscéral entourant les organes. L'afflux sanguin y est très important du fait du grand nombre de vaisseaux et capillaires sanguins, notamment au niveau du feuillet pariétal. La surface du réseau vasculaire représente environ 1 m². Entre les deux feuillets, se loge un espace virtuel : la cavité péritonéale. Pour effectuer la dialyse, un liquide artificiel, le dialysat, est introduit dans la cavité péritonéale. Ce liquide sera ensuite évacué après un temps de contact déterminé.

Les dialysats les plus couramment utilisés sont composés d'une solution tampon (du lactate ou du bicarbonate) à pH acide (5,2 - 5,5) ou physiologique (7,4) à laquelle est ajoutée des électrolytes (sodium, calcium, magnésium, chlore) et un agent osmotique (du glucose ou un polymère de glucose, tel que l'« icodextrine » présent dans la solution pour dialyse péritonéale ambulatoire EXTRANEAL® commercialisée par la société BAXTER).

Les électrolytes et l'agent osmotique jouent chacun un rôle dans le mécanisme d'échange, selon leurs propriétés physico-chimiques respectives :
- les déchets du métabolisme (tels que l'urée ou la créatinine) ou autres électrolytes en surabondance que le rein n'élimine plus ou insuffisamment via l'appareil urinaire et les urines, vont s'extraire du plasma sanguin par diffusion des éléments vers le dialysat dont les taux de concentration de ces mêmes éléments sont moindres ;
- l'excédent d'eau, que le rein élimine normalement pour la régulation du volume plasmatique, va être attiré par osmolarité ; ce processus est nommé ultrafiltration ; le taux d'ultrafiltration varie en fonction de la concentration du dialysat en glucose ou en polymère de glucose : plus la solution sera concentrée, plus l'eau présente dans le corps sera captée par le dialysat.

Cependant, bien que le glucose ait l'avantage d'être relativement sûr et peu coûteux, il a un certain nombre d'inconvénients. En raison du sa petite taille, le glucose traversant rapidement le péritoine mène à la perte de gradient osmotique et à la perte d'ultrafiltration dans les 2 à 4 heures d'infusion.

Par ailleurs, l'introduction quotidienne du dialysat peut provoquer à terme un risque d'altération de la membrane péritonéale, contraignant l'emploi de cette méthode pour une durée limitée dans le temps, généralement entre deux et dix ans.

Le cathéter implanté dans la cavité péritonéale est une porte d'entrée propice aux germes. Les nombreuses manipulations sur le cathéter lors des phases d'infusion et de drainage augmentent le risque d'infection locale ou générale

Il a été suggéré que les caractéristiques d'ultrafiltration des solutions de dialyse péritonéale pourraient être meilleures en remplaçant le glucose par des substances de haut poids moléculaire, telles que des polymères de glucose.

Les polymères de glucose standards sont produits par hydrolyse acide ou enzymatique d'amidon de céréales ou de tubercules.

L'hydrolyse acide de l'amidon, totalement aléatoire, ou son hydrolyse enzymatique un peu plus ordonnée, fournissent des mélanges de glucose et de polymères du glucose qui comportent des molécules très courtes, de faible Degré de polymérisation (ou D.P.), aussi bien que des molécules très longues, de D.P. élevé. Les polymères de glucose ont ainsi un poids moléculaire extrêmement varié.

Dans le domaine plus particulier de l'utilisation des polymères du glucose destinés à la dialyse péritonéale continue et ambulatoire, la demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

On conçoit en effet aisément pour cette application que les monomères ou polymères de faible poids moléculaire traversent rapidement la paroi péritonéale et sont ainsi sans intérêt durable pour la création d'un gradient de pression osmotique, et que les polymères de très haut poids moléculaire, dénués de pouvoir osmotique, sont à éviter et même à proscrire puisque potentiellement dangereux s'il leur advenait de précipiter consécutivement à leur rétrogradation.

Les procédés proposés dans cette demande de brevet EP 207.676 pour obtenir ces polymères de glucose de faible indice de polymolécularité consistent :
- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Soucieuse de mettre au point un procédé de fabrication d'un hydrolysat d'amidon complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse, s'est attachée à résoudre ce problème dans son brevet EP 667.356.

Ce procédé consiste à :
- hydrolyser par voie acide un lait d'amidon waxy (ou cireux) jusqu'à un D.E. compris entre 8 et 15 ;
- éventuellement compléter cette hydrolyse acide par une hydrolyse enzymatique à l'aide d'alpha-amylase bactérienne jusqu'à un D.E. compris entre 11 et 18 ;
- chromatographier sur résines cationiques fortes macroporeuses sous forme alcaline ou alcalino-terreuse cet hydrolysat double acide-enzyme ;
- collecter l'hydrolysat d'amidon exclu lors de cette étape de chromatographie.

Dans ce brevet, pour obtenir un hydrolysat d'amidon présentant un indice de polymolécularité inférieur à 2,5, il est collecté l'hydrolysat d'amidon exclu lors de cette étape de chromatographie dans un rendement pondéral de l'ordre de 60 % de l'hydrolysat mis en oeuvre à l'étape de chromatographie.

Cet hydrolysat d'amidon en question contient alors de préférence moins de 3 % de glucose et de polymères de glucose de DP inférieur ou égal à 3 et moins de 0,5 % de polymères de glucose de DP supérieur à 600.

Il est admis par les experts du domaine de la dialyse péritonéale que ces polymères de glucose, utilisés pour leur pouvoir osmotique, donnent toute satisfaction.

Il n'en reste pas moins que le traitement par dialyse péritonéale présente un certains nombre d'inconvénients liés aux risques du procédé.

Un des risques majeurs est la péritonite.

Le soupçon clinique de la péritonite est diagnostiqué lors du développement d'un trouble dans le dialysat associé avec les manifestations cliniques variables que sont la douleur abdominale, la nausée, le vomissement, la diarrhée et la fièvre.

Ces épisodes de la péritonite sont provoqués par des infections bactériennes intrapéritonéales, et le diagnostic est habituellement facilement établi par les cultures positives de dialysat.

La « péritonite stérile », également décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéales, des cas sporadiques de péritonite aseptique ont été rapportés pouvant être liées à des causes diverses et notamment l'induction par des substances pro-inflammatoires potentiellement présentes.

Par ailleurs, les tests décrits aujourd'hui dans les Pharmacopées pour la détection de substances pyrogènes sont les suivants :
- Le test de détection des endotoxines bactériennes, composants majoritaires des bactéries Gram négatives (test LAL),
- Le test pyrogène lapin.

Bien que généralement fiables, ces deux tests présentent leurs limites.

Le test pyrogène lapin est fondé sur la détection indirecte de substances pyrogènes par la mesure d'une élévation de température du lapin auquel a été injecté le produit contenant ces substances (réponse fébrile).

Ce test peut donner lieu à des faux négatifs, si la substance indésirable présente une activité biologique trop faible ou une concentration trop basse pour induire une réponse systémique pyrogène.

Cependant, cette substance peut posséder une activité biologique ou concentration suffisante pour produire une réaction inflammatoire locale.

Le test LAL quant à lui ne détecte que les endotoxines bactériennes (LPS) ainsi que les β glucanes, composants des parois de flores fongiques.

Les autres impuretés biologiques (ADN,...) ne sont pas détectées. Il en est de même pour les peptidoglycanes, composants majoritaires des membranes cellulaires des bactéries Gram positives.

La manifestation des péritonites aseptiques observées avec les solutions de dialyse péritonéale contenant de l'icodextrine témoigne donc, pour certains cas, de la façon dont certaines substances peuvent échapper aux tests décrits dans les pharmacopées et peuvent être à l'origine d'effets cliniques indésirables.

Pour remédier à cette situation, la société BAXTER propose de placer les efforts dans la détection des contaminants microbiens Gram positifs.

En particulier, dans son brevet EP 1.720.999, la société BAXTER propose de développer une méthode basée sur la détection des peptidoglycanes, qui sont les composants majeurs des membranes des bactéries Gram positives, notamment dans les polymères de glucose destinés à la préparation de solution pour dialyse péritonéale.

Cette méthode consiste à réaliser sur les polymères de glucose :
- un essai de « biocharge » pour détecter un microorganisme Gram positif thermophile acidophile particulier : *Alicyclobacillus acidocaldarius,* puis
- une stérilisation desdits polymères de glucose, puis
- un test consistant à ajouter un réactif capable de réagir avec les peptidoglycanes pour induire une réaction en cascade de sérine-protéase,
- une quantification desdits peptidoglycanes.

S'il est déterminé que la quantité de ces peptidoglycanes recherchée dans les polymères de glucose est inférieure à un certain seuil (10 ng/ml d'une solution à 7,5% de polymère de glucose, soit 133 ng/g de polymères de glucose), ces polymères de glucose sont alors utilisés pour préparer la solution de dialyse péritonéale proprement dite.

En d'autres termes, pour empêcher la survenue de ces épisodes de péritonites aseptiques, la société BAXTER propose pour la fabrication et l'usage des solutions de dialyse péritonéale, un protocole de détection de peptidoglycanes dans la solution de dialyse péritonéale.

Par ailleurs, s'il est évoqué dans ce brevet EP 1.720.999 le traitement amont des polymères de glucose, il ne l'est qu'au moyen de résines d'affinités susceptibles de piéger les peptidoglycanes en tant que tels.

Il n'est donc pas envisagé de modifier le procédé de fabrication des polymères de glucose de telle sorte que le produit final soit dépourvu de contamination par des bactéries acidothermophiles de type *Alicyclobacillus acidocaldarius* ou par des débris membranaires de ces bactéries particulières.

Par conséquent, il existe un besoin non satisfait de fournir, par un procédé de préparation et de purification sécurisé, des substances destinées à la dialyse péritonéale de meilleure qualité, en l'occurrence des polymères de glucose, afin d'assurer que ces substances sont efficacement dépourvues de substances contaminantes.

La société Demanderesse a donc trouvé que ce besoin pouvait être satisfait par la mise en oeuvre d'un procédé de purification remarquable, combinant un certain nombre d'étapes de traitement au charbon actif / noir granulaire, de filtration (microfiltration et ultrafiltration) et de traitement thermique dans un agencement propre à empêcher toute contamination.

Le procédé de purification de polymères de glucose destinés à la fabrication de solutions de dialyse péritonéale conforme à l'invention est caractérisé en ce qu'il comprend :
- au moins une étape de traitement au charbon actif et/ou au noir granulaire,
- au moins une étape de filtration stérilisante consistant en deux filtrations membranaires d'un diamètre de pore de 0,45 µm puis 0,22 µm,
- au moins une étape de traitement thermique consistant à chauffer à une température comprise entre 100 et 130°C pendant 1 à 5 minutes, et
- au moins une étape d'ultrafiltration, la membrane d'ultrafiltration présentant un seuil de coupure compris entre 30.000 et 100.000 daltons.

Il est important de noter que la combinaison de ces quatre étapes garantissent la quasi-absence de contaminants de toute nature quelle que soit leur taille (e.g. endotoxines, peptidoglycanes et β-glucanes).

Ce procédé est appliqué sur les polymères de glucose destinés à la fabrication de solutions de dialyse péritonéale finis, c'est-à-dire ceux qui seront utilisés pour la préparation de la solution de dialyse.

Le caractère sécurisé d'un tel procédé permet plus particulièrement de limiter les contrôles bactériens au terme dudit procédé au test de haute sensibilité de détection des peptidoglycanes mis au point et validée par la société Demanderesse (qui sera décrit ci-après), et au test de détection des endotoxines (test LAL).

Par « quasi-absence », on entend au sens de l'invention une quantification à des seuils bien inférieurs à ce qui est décrit dans les tests de Pharmacopées, i.e. :
- pour les endotoxines (et β glucanes) via le test LAL (méthode gel clot en point final) utilisant des réactifs fabriqués par CHARLES RIVER-ENDOSAFE (Lysat LAL de sensibilité 0,015 E.U/ml réf. OR15015 et endotoxines CSE 500 ng ou 10 ng par flacon réf.E110 ou E120) : ≤ 0.6 EU/g
- pour les peptidoglycanes (et β-glucanes) via un test de haute sensibilité mis au point par la société Demanderesse : < 8 ng/g de polymères de glucose (ainsi bien inférieur au seuil de référence décrit dans le brevet EP 1.720.999 pour les peptidoglycanes).

Par « test de haute sensibilité mis au point et validé par la société Demanderesse », on entend un test développé et validé par la société Demanderesse, en adaptant le kit SLP-HS single set ref. 293-58301 fabriqué et commercialisé par la société WAKO Pure Chemical Industries Ltd.

Ce test consiste à additionner le réactif dit « SLP-HS » (Silkworm Larvea Plasma-High sensitivity) réactif préparé à partir du plasma de ver à soie, capable de :
- réagir avec les peptidoglycanes et β-glucanes contenus dans une solution de polymère de glucose préparée à 5 % dans de l'eau (eau spéciale pour test LAL par exemple),
- induire une réaction en cascade de sérine-protéase et
- de détecter et/ou de quantifier lesdits peptidoglycanes et β-glucanes au moyen d'un lecteur de tubes TOXINOMETER fabriqué et commercialisé par la société WAKO Pure Chemical Industries Ltd à des seuils très faibles, i.e. :
   - une limite de détection (LD) à un seuil d'environ 0,05 ng/ml (soit 1 ng/g de polymère de glucose) et
   - une limite de quantification (LQ) à un seuil d'environ 0,15 ng/ml (3 ng/g de polymère de glucose).

### (LD et LQ déterminées dans le produit polymère de glucose testé)

De manière plus précise, le test SLP-HP consiste à :
- préparer le polymère de glucose à tester en solution à 5 % dans de l'eau de qualité adéquate (eau spéciale pour test LAL par exemple),
- réaliser une gamme étalon de peptidoglycanes dans l'eau sur le domaine d'application de 0,04 à 2,5 ng/ml (valeurs cibles) avec le standard de peptidoglycanes (extrait de *Staphylococcus Aureus*) du kit SLP-HS single set pour l'établissement d'une droite d'étalonnage (régression linéaire en échelle logarithmique Ta = f(teneur en PG)),
- introduire 100 µl de la solution préparée à tester dans le tube HS-SLP après reconstitution par ajout de 100 µl du diluant (fourni dans le kit précédemment cité),
- introduire le tube SLP-HS dans le puit d'incubation du lecteur de tube TOXINOMETER (WAKO Pure Chemical Ltd) thermostaté à 30°C et paramétré selon les conditions prescrites par le fabricant,
la teneur en PG de la solution à tester est calculée au moyen de la droite d'étalonnage établie.

Le résultat est exprimé en ng/ml de solution à 5 % testée puis en ng/g de polymère de glucose.

Il est remarquable que les quantités de ces peptidoglycanes/ β glucanes dans le polymère de glucose au final obtenu grâce au procédé conforme à l'invention sont garanties bien inférieures à 8 ng/g de polymère de glucose, soit au minimum et environ 16 fois inférieur au seuil décrit dans le brevet EP 1.720.999.

Dans le procédé de purification des polymères de glucose destinés à la dialyse péritonéale, comme il a été décrit plus haut, le premier des quatre moyens mis en oeuvre consiste à utiliser du charbon actif et/ou du noir granulaire dans une configuration particulière.

La société Demanderesse recommande de mettre en oeuvre ce moyen dans au moins une des trois variantes suivantes :
- en première variante du procédé conforme à l'invention : dans le cas de l'utilisation du noir granulaire, cette configuration repose sur un fonctionnement à contre-courant.

Le temps de séjour dans la colonne est d'environ trois heures. La percolation se fait à une vitesse de l'ordre de 2 m/h à une température de l'ordre de 80°C pour éviter la contamination bactérienne.

Le contact entre l'hydrolysat d'amidon à purifier avec le noir granulaire se fait à contre-courant au sens où l'hydrolysat d'amidon à purifier entre tout d'abord en contact avec le noir granulaire saturé en bas de colonne.

L'hydrolysat d'amidon purifié est donc récupéré au sommet de la colonne de noir granulaire, en même temps que le noir granulaire purifié.

De cette manière, la dernière couche de noir granulaire en haut de colonne sert de « barrière de sécurité ».

On peut contrôler cet arrangement en mettant en oeuvre des opérations de « chasse » de noir granulaire. La colonne est stoppée, on soutire par le bas le noir granulaire saturé, que l'on remplace par le haut avec du noir granulaire régénéré.

Le noir granulaire saturé est désucré avant d'être régénéré par traitement thermique en four à sole.

Au démarrage, et par sécurité, les premiers m³ d'hydrolysat d'amidon de basses matières sèches sont déclassés.

Le suivi de l'abaissement du taux de contaminants (endotoxines, peptidoglycanes et β-glucanes) peut être analysé en faisant un certain nombre de prélèvements (cinq par exemple) du bas de la colonne vers le haut.
- en deuxième variante du procédé conforme à l'invention : dans le cas de l'utilisation du charbon actif, cette configuration repose sur un traitement au charbon actif « en double ».

L'hydrolysat d'amidon entrant est mélangé avec du charbon actif (entre 0,5% et 1,5 % sur la matière sèche à traiter) à une température comprise entre 70 et 75°C pendant une heure.

L'hydrolysat d'amidon est ensuite filtré et analysé.

L'hydrolysat d'amidon subit alors un traitement de même nature. Ce deuxième traitement est le traitement dit « de sécurité ».

La société Demanderesse recommande d'utiliser dans ces deux étages du charbon actif de porosité différente, de manière à prendre en compte la variabilité de la taille des contaminants.
- en troisième variante du procédé conforme à l'invention, il est choisi de combiner un étage de noir granulaire et un étage de charbon actif.

La société Demanderesse recommande alors de placer la colonne de noir granulaire en tête de cette combinaison.

Les conditions de mise en oeuvre de ces deux étages sont conformes à ce qui est décrit ci-avant.

Le deuxième des quatre moyens mis en oeuvre pour purifier les polymères de glucose destinés à la fabrication de solutions de dialyse péritonéale conforme à l'invention consiste à utiliser une filtration stérilisante.

Cette étape permet de retenir toute contamination par des microorganismes, et notamment les bactéries acidothermophiles de type *Alicyclobacillus acidocaldarius*, leur taille étant supérieure aux diamètres des pores de filtration.

La filtration est réalisée par plusieurs filtres à cartouches insérées dans un carter vertical vers lequel le sirop est dirigé. Ces filtres à cartouches sont fournis par les sociétés PALL ou MILLIPORE par exemple. La taille des cartouches peut être de 10, 20 ou 30 pouces, et le nombre de cartouches installées permet d'obtenir une surface de filtration suffisante afin de passer un débit de produit entre 1 et 20 1/minutes/m².

Ces filtres à cartouches ont des capacités de résistance pour un travail en continu à haute température, de l'ordre de 75°C et pour passer le débit précédemment cité durant une période supérieure de 700 h.

Le fait de travailler à une température supérieure à 75°C permet de limiter tout développement microbiologique, notamment des flores thermophiles.

Leur résistance à la température permet également de réaliser une stérilisation avant leur mise en service. Cette stérilisation consiste à faire passer de la vapeur 2 bars à travers le carter pendant une période de 20 minutes. Cette stérilisation est suivie par un rinçage à l'eau purifiée (au sens de la Pharmacopée) pendant une période de 5 minutes.

Ces filtres possèdent également des capacités de résistance à certains produits chimiques utilisés pour les opérations de nettoyage des équipements, et notamment l'acide peracétique à une concentration de 5‰.

Un test d'intégrité est réalisable sur ces cartouches à l'aide d'un intégritest de la société MILLIPORE par exemple. Ce test d'intégrité est réalisé à la mise en place des cartouches afin d'en vérifier le montage. Ce test est ensuite réalisé avant chaque nettoyage des équipements et enfin avant le démontage afin de valider leur bon fonctionnement durant la phase de production.

Le delta de pression (ΔP) de travail de ces filtres ne doit pas dépasser les 2 bars afin de garantir leur intégrité. Si tel est le cas, ces filtres doivent être remplacés par de nouveaux.

Le troisième des quatre moyens mis en oeuvre pour purifier les polymères de glucose destinés à la fabrication de solutions de dialyse péritonéale conforme à l'invention consiste à utiliser un traitement thermique.

Plus particulièrement, un traitement thermique dont le couple temps / température est choisi de manière à réduire la biocharge en microorganismes thermophiles susceptibles de contaminer les polymères de glucose.

Cette étape de traitement thermique consiste alors à chauffer à une température comprise de préférence à une température de 120°C, pendant de préférence 2 minutes.

Cette étape de traitement à température élevée pendant une courte durée n'a aucune commune mesure avec les heures de traitement thermique classiquement réalisé dans l'état de la technique par ébullition de milieu réactionnel afin par exemple de dénaturer les protéines (notamment pour inactiver les enzymes).

Le traitement thermique selon l'invention est réalisé à l'aide d'un échangeur tubulaire dans lequel l'hydrolysat d'amidon chromatographié circule, et est entouré d'une calandre alimentée par de la vapeur 2 bars afin d'y réguler une température de l'ordre de 120°C.

Cet échangeur tubulaire étant par exemple fabriqué par la société ACTINI, est constitué de plusieurs parties :
- une section de récupération d'énergie produit/produit entre l'entrée et la sortie de la zone
- une section de chauffage à l'aide de vapeur 2 bars
- une section de chambrage intégrée et modulable en fonction du temps de séjour souhaitée.

La longueur de cet échangeur est calculée afin de garantir le temps de séjour souhaité suivant le débit d'alimentation. Par exemple le débit d'alimentation peut être compris entre 3000 et 4000 litres/heure pour une section de chambrage de l'ordre de 100 à 130 litres.

Ce traitement thermique permettant une réduction de d'au moins 2 log sur les microorganismes, et notamment les bactéries acidothermophiles de type *Alicyclobacillus acidocaldarius.*

Le quatrième des quatre moyens mis en oeuvre pour purifier les polymères de glucose destinés à la fabrication de solutions de dialyse péritonéale conforme à l'invention consiste à utiliser une ultrafiltration.

Plus particulièrement, le seuil de coupure est choisi de manière à retenir les contaminants éventuels dans le rétentat.

La membrane d'ultrafiltration présente alors un seuil de coupure de préférence de l'ordre de 50.000 daltons.

La surface du filtre détermine la capacité de filtration du filtre. Cette surface étant déterminée en fonction de la nature du fluide et du débit à traiter.

Le seuil de coupure permet de retenir les microorganismes, et notamment les bactéries acidothermophiles de type *Alicyclobacillus acidocaldarius,* ainsi qu'une partie des endotoxines, peptidoglycanes et β-glucanes, leur taille étant comprise entre 1.000 et 100.000 Daltons.

Les membranes d'ultrafiltration peuvent être de type céramique ou organique. Ces deux types de membranes ayant une résistance différente à la température, il sera préféré les membranes de type céramique qui permettent de travailler à des températures supérieures à 75°C.

Leur résistance à la température permet de réaliser une stérilisation à la vapeur avant leur mise en service. Cette stérilisation consiste à faire passer de la vapeur 2 bars à travers le carter pendant une période de 20 minutes. Cette stérilisation est suivie par un rinçage à l'eau purifiée pendant une période de 20 minutes.

Il convient ainsi également de travailler à une température supérieure à 75°C pour éviter tout développement microbiologique.

Ces filtres possèdent également des capacités de résistance à certains produits chimiques utilisés pour le nettoyage des équipements, et notamment l'acide peracétique à une concentration de 5‰ et la soude à une concentration de 1 %.

La pression du sirop d'alimentation est comprise entre 5 et 10 bars et régulée par la pompe alimentant ce module. Dans le cas où la pression maximale est atteinte mais que le débit de sirop est trop faible, il convient de réaliser un nettoyage à la soude des membranes afin qu'elles retrouvent une pleine efficacité.

Le suivi de l'abaissement du taux de contaminants (endotoxines, peptidoglycanes et β-glucanes) peut être analysé en faisant des prélèvements périodiques sur le filtrat.

Ce quatrième moyen permet avec les trois autres moyens de garantir dans le produit final la présence d'un éventuel contaminant de type peptidoglycanes, endotoxines et/ou β-glucanes à une valeur inférieure aux seuils définis ci-avant.

Un des procédés conformes à l'invention préféré pour l'obtention de polymères de glucose destiné à la préparation de solution pour dialyse péritonéale peut être ainsi détaillé par la succession des étapes suivantes :
1) obtenir un lait d'amidon waxy d'une matière sèche finale comprise entre 35 et 40 %,
2) hydrolyser par voie acide le lait d'amidon waxy ainsi obtenu et éventuellement, compléter cette hydrolyse acide par une hydrolyse enzymatique à l'aide d'alpha-amylase bactérienne jusqu'à un D.E. compris entre 9 et 14, de préférence entre 10 et 13,
3) réaliser sur l'hydrolysat d'amidon ainsi obtenu une étape de traitement au charbon actif et/ou au noir granulaire,
4) mettre en oeuvre une filtration stérilisante consistant en deux filtrations membranaires de diamètre de pore de 0,45 µm puis 0,22 µm,
5) chromatographier sur résines cationiques fortes macroporeuses sous forme alcaline ou alcalino-terreuse cet hydrolysat ;
6) collecter l'hydrolysat d'amidon, plus précisément les polymères de glucose, exclu lors de cette étape de chromatographie,
7) mettre en oeuvre sur ces polymères de glucose une étape de traitement thermique à une température de 120°C pendant 2 minutes,
8) réaliser une étape de traitement au charbon actif et/ou au noir granulaire,
9) mettre en oeuvre une filtration stérilisante consistant en deux filtrations membranaires de diamètre de pore de 0,45 µm puis 0,22 µm,
10) réaliser une ultrafiltration d'un seuil de coupure compris entre 30.000 et 100.000 daltons, de préférence de l'ordre de 50.000 daltons.

Il apparaît que cette succession d'étapes permet de sécuriser, pas à pas, l'obtention des polymères de glucose destinés à la préparation de solution pour dialyse péritonéale.

Une première étape de traitement au charbon actif et/ou au noir granulaire est effectuée sur l'hydrolysat d'amidon.

Une étape de filtration stérilisante est effectuée sur l'hydrolysat d'amidon traité sur charbon actif et/ou au noir granulaire, avant son passage sur chromatographie.

Enfin, l'hydrolysat d'amidon chromatographié subit quatre étapes de purification successive :
- traitement thermique,
- charbon actif et/ou noir granulaire,
- filtration stérilisante,
- ultrafiltration.

Ces polymères de glucose sont alors utilisés pour préparer la solution de dialyse péritonéale proprement dite.

L'invention sera mieux comprise à l'aide des exemples qui suivent, lesquels se veulent illustratifs et non limitatifs.

### Exemple de référence 1 :

La matière première pour l'obtention des polymères de glucose selon l'invention est produite à partir d'amidon de maïs waxy de la manière suivante :
- nettoyage du maïs de manière à garder exclusivement les grains de maïs entier,
- trempe du maïs ainsi nettoyé en présence d'acide lactique de manière à assouplir les grains,
- broyage humide, puis séparation des différents constituants, i.e. germe, enveloppe cellulosique, protéines et amidon,
- nettoyage de l'amidon à contre courant avec de l'eau sanitisée de manière à purifier l'amidon aussi bien physico-chimiquement que bactériologiquement,
- centrifugation et séchage de l'amidon,
- mise en suspension de l'amidon dans une eau sanitisée à une matière sèche finale de 40 % et à une Température de 45°C à 50°C,
- acidification de la suspension d'amidon par addition d'HCl à un pH < 2, et accroissement de la température jusqu'à 115 à 120°C pendant 6 à 8 minutes,
- floculation des protéines et des matières grasses à ce pH,
- neutralisation de la suspension à pH 5,
- filtration de la suspension sur terre de diatomées (de manière à retenir les protéines, les matières grasses et la cellulose résiduelles),
- déminéralisation sur résine cationique forte et résine anionique faible,
- traitement au charbon actif à une Tp de 70-80°C et à un pH de 4 à 4,5 ; ce qui élimine les impuretés colorées et réduit le niveau d'impuretés microbiologiques.

Le charbon actif poudre étant ajouté à une concentration comprise entre 0,2 et 0,5% sur sec est retenu sur un filtre céramique de 10 µm chargé auparavant avec un agent filtrant,
- concentration par passage sur évaporateur à film tombant,
- atomisation de la solution concentrée dans un atomiseur de type MSD commercialisée par la société NIRO.

Cet hydrolysat d'amidon est conforme à la monographie de la pharmacopée européenne (réf Maltodextrines : 1542).
∘ pH : 4,0 - 7,0 pour une solution à solution 10 %,
∘ I. d. : conforme,
∘ Perte à la dessiccation : 6 % max
∘ DE : < 20
∘ Cendres sulfuriques : 0,5 % max
∘ SO₂ : 20 ppm max
∘ Métaux lourds : < 10 ppm
∘ *E. coli* : absent / g
∘ Salmonelles : absent / 10 g
∘ Germes viables totaux : 100 CFU/g max (EP 1000 CFU/g)
∘ Moisissures : 100 CFU/g max

En complément, nous analysons les lots produits sur les valeurs de :
- contamination en levures + moisissures : 150 CFU/10 g max, soit 15 / g max
- germes aérobies : 500 CFU/10 g max, soit 50/g max
- Endotoxines (test LAL gel clot en point final) : 20 EU/g max
- Peptidoglycanes : (test validé SLP-HS) : 2700 ng/g max

Les conditions d'obtention des polymères de glucose conformes à l'invention à partir de l'hydrolysat d'amidon ainsi obtenu sont les suivantes :
1) Préparation de l'eau / Qualité de l'eau
   - purification de l'eau par filtration sur 3 µm ; traitement sur charbon actif, déminéralisation sur résines échangeuses de cations et d'anions, et filtration à nouveau (UA),
   - deux réservoirs utilisés :
      ∘ 10 m³ pour la dissolution de l'hydrolysat d'amidon, les étapes de rinçage et nettoyage de l'atomiseur,
      ∘ 60 m³ pour le process principal (nettoyage des réservoirs, ses suspensions de charbon actif et de la chromatographie
2) Chromatographie
   - solubilisation de l'hydrolysat d'amidon avec de l'eau purifiée de manière à obtenir une MS de 35 - 45 % à une température comprise entre 60 - 85°C,
   - filtration stérilisante de l'hydrolysat d'amidon par passage sur 0,45 µm puis 0,22 µm, réalisée à un ΔP < 3 bars,
   - séparation chromatographique par exclusion stérique (SEC) réalisée à l'aide d'un système continu composé de 6 séries de double plateaux de 1 m³ de résine chacun. La résine mise en oeuvre est une PCR145K commercialisée par la société Purolite.

La solution qui traverse cette résine présente une température comprise entre 75 et 85°C à 35-45 % de MS.

La durée de chaque séquence définit le process.

Dans le cas présent, la durée de chaque séquence est de 15 minutes.

Le contrôle est effectué par une analyse de distribution du poids moléculaire et l'analyse du rendement de chromatographie, de la manière suivante : (Quantité de matière sèche de la fraction voulue)/ (Quantité de matière sèche de l'alimentation)

Les poids moléculaires les plus faibles interagissent avec la résine et les haut poids moléculaires sont élués à l'eau purifiée.
- concentration est effectuée par évaporation en film tombant à une MS de 35 - 45 %.
- réalise un traitement thermique à une température de 120°C pendant 2 minutes,
- ajout de charbon actif entre 0,5 et 1,5 % de la masse totale de l'hydrolysat d'amidon à 75°C avec des résines cationiques (1 à 3 l) pour contrôler le pH (4 - 4,5) et anioniques (5 à 10 l) pour contrôler le pH (5,5 - 6),
- filtration réalisée sur filtres à manches en polypropylène avec ΔP < 5 bars, en 5 à 6 heures par lot.
- seconde et troisième filtration sur 1,5 et 0,45 µm sont ensuite effectuées
- puis sur 0,22 et 0,1 µm,
- ultrafiltration sur membrane d'un seuil de coupure de l'ordre de 40.000 Da

Pour l'atomisation : alimentation à 500 kgs /h avec une solution à 40% de MS et à 250°C dans un atomiseur de type MSD commercialisé par la société Niro.

Le produit atomisé présente à sa sortie une humidité inférieure à 6 %.

On refroidit alors le produit en lit d'air fluidisé comprenant 3 zones de refroidissement alimentée par de l'air à 40, 30 et 20°C. Le produit obtenu est ensuite tamisé sur 800µm afin de retirer les agrégats.

De l'ordre de 500 kgs de produit fini sont obtenus à partir de 800 kgs de maltodextrines de départ, soit un rendement de l'ordre de 60%.

La détermination de la contamination éventuelle du circuit est réalisée par l'analyse de la teneur en peptidoglycanes et endotoxines sur le produit fini.

Pour exemple, les teneurs habituellement observées et mesurées sur les lots du produit fini (exprimés par g de polymère de glucose) sont pour les critères spécifiés ci-dessus les suivants :
Levures et moisissures : 0 / g
Germes aérobies : 0/g
Endotoxines (test LAL gel clot en point final) : ≤ à 0.3 EU/g.
Peptidoglycanes (test validé SLP-HS) : < 3 ng/g
*B. acidocaldarius* : 1 /g

### Exemple 2

Il est décidé de tester l'efficacité du procédé de purification de polymères de glucose, destinés à la fabrication de solutions de dialyse péritonéale, conforme à l'invention (i.e. caractérisés par la mise en oeuvre de chacune des 4 étapes de traitement), par rapport à un procédé de purification n'utilisant que deux, voire trois des quatre étapes du procédé conforme à l'invention (une étape de traitement au charbon actif et une étape de filtration stérilisante dans un premier cas de figure, une étape de traitement au charbon actif, une étape de filtration stérilisante et une étape de traitement thermique dans un deuxième cas de figure).

### 1) mise en oeuvre du procédé de purification en deux étapes :

Les détails des conditions opératoires sont ceux définis dans l'exemple 1 à l'exception des étapes de traitement thermique et d'ultrafiltration non utilisés dans ce premier cas.

On choisit un lot « A » de polymères de glucose dont les analyses microbiologiques ont donné les résultats suivants :

| | |
|---|---|
| Levures et moisissures : | < 50 / 10 g |
| Germes aérobies : | 50 / 10 g |
| Endotoxines : | 19 EU/g (test LAL gel clot en point final) |
| Peptidoglycanes : | 1520 ng/g (test validé SLP-HS) |
| *B. acidocaldarius* : | 1 / g |

Ce procédé utilise du charbon actif en poudre de marque NORIT SX+ à 0,65 % sec/sec et le traitement est appliqué durant un temps de contact de 1 heure.

On applique ensuite une étape de filtration stérilisante avec des filtres cartouche en série commercialisés par la société MILLIPORE de 0,45 µm et de 0,22 µm.

Le produit fini « B » présente alors les résultats microbiologiques suivants :

| | |
|---|---|
| Levures et moisissures : | 0 / g |
| Germes aérobies : | 0 / g |
| Endotoxines : | < 0,3 EU/g (test LAL gel clot en point final) |
| Peptidoglycanes : | 47 ng/g (test validé SLP-HS) |
| *B. acidocaldarius* : | 0 / g |

### 2) mise en oeuvre du procédé de purification en trois étapes

On applique au produit fini « B » les conditions opératoires suivantes :
- préparation d'une solution à 10 % de matière sèche avec de l'eau purifiée,
- traitement thermique à 120°C pendant 2 minutes
- concentration à 30 % de matière sèche

Le produit fini « C » présente alors les résultats microbiologiques suivants :

| | |
|---|---|
| Levures et moisissures : | 0 / g |
| Germes aérobies : | 0 / g |
| Endotoxines : | < 0,3 EU/g (test LAL gel clot en point final) |
| Peptidoglycanes : | < 20 ng/g (test validé SLP-HS) |
| *B. acidocaldarius* : | 0 / g |

### 3) mise en oeuvre du procédé de purification conforme à l'invention

On applique au produit fini « C » les conditions opératoires suivantes :
- ultrafiltration sur une membrane de seuil de coupure de 40.000 daltons

Le produit fini « D » présente alors les résultats microbiologiques suivants :

| | |
|---|---|
| Levures et moisissures : | 0 / g |
| Germes aérobies : | 0 / g |
| Endotoxines : | < 0,3 EU/g (test LAL gel clot en point final) |
| Peptidoglycanes : | < 3 ng/g (test validé SLP-HS) |
| *B. acidocaldarius* : | 0 / g |

Il apparaît clairement que le procédé de purification conforme à l'invention permet de garantir un taux de contaminant en peptidoglycanes remarquablement bas; le procédé de purification en deux étapes et en trois étapes ne permettant pas, à partir du lot « A » de récupérer un polymère de glucose présentant même un taux de contaminant en peptidoglycanes inférieur à la valeur seuil de 8 ng/g qui en autoriserait l'utilisation en dialyse péritonéale.

Il est ainsi prouvé que même s'il advenait qu'un des lots de polymères de glucose se révélait anormalement chargé en peptidoglycanes, le procédé de purification conforme à l'invention permettrait d'en réduire efficacement le contenu, garantissant ainsi une teneur en contamination bien inférieure à la limite de tolérance communément admise.

### Exemple 3 (de référence)

Pour garantir la qualité des circuits mis en oeuvre, on procède au lavage régulier des équipements de la manière suivante.

On choisit ici le nettoyage d'une cuve de dissolution de matière première (hydrolysat d'amidon).

Après utilisation de cette cuve pour la production de 12 lots de produit fini, nous appliquons les séquences suivantes:
- lavage à l'eau par un système de nettoyage en place (NEP) afin de retirer toute trace de produit (hydrolysat d'amidon).
   L'efficacité de ce lavage est contrôlée par une mesure de lecture réfractométrique (LR) sur les eaux de lavage en sortie de cette cuve (LR<0.5)
- introduction de soude du fabricant Solvay à 1% par le même système de NEP pendant 30 minutes.
- rinçage à l'eau pour éliminer les traces de soude.
   L'efficacité de ce rinçage est contrôlée par une mesure de pH sur les eaux de rinçage en sortie de cette cuve.
- introduction d'acide peracétique Bactipal de la société SEPPIC dilué à 0.05% par le même système de NEP pendant 10 minutes.
- rinçage à l'eau purifiée pour éliminer les traces d'acide.

l'efficacité de ce rinçage est contrôlée par un test peroxydes sur les eaux de rinçage en sortie de cette cuve.

## Revendications

1. Procédé de purification de polymères de glucose destinés à la fabrication de solutions de dialyse péritonéale, **caractérisé en ce qu'**il comprend :
- au moins une étape de traitement au charbon actif et/ou au noir granulaire,
- au moins une étape de filtration stérilisante consistant en deux filtrations membranaires d'un diamètre de pore de 0,45 µm puis 0,22 µm,
- au moins une étape de traitement thermique consistant à chauffer à une température comprise entre 100 et 130°C pendant 1 à 5 minutes, et
- au moins une étape d'ultrafiltration, la membrane d'ultrafiltration présentant un seuil de coupure compris entre 30.000 et 100.000 daltons.

2. Procédé selon la revendication 1, **caractérisé en ce que** l'étape de traitement thermique consiste à chauffer à une température de 120°C pendant 2 minutes.

3. Procédé selon l'une quelconque des revendications 1 à 2, **caractérisé en ce que** le traitement au charbon actif ou au noir granulaire consiste en deux étages, le premier composé de charbon actif, le second composé de charbon actif ou de noir granulaire.

4. Procédé selon l'une quelconque des revendications 1 à 3, **caractérisé en ce que** la membrane d'ultrafiltration présente un seuil de coupure de l'ordre de 50.000 daltons.

5. Procédé selon la revendication 1, **caractérisé en ce qu'**il consiste à :
1) obtenir un lait d'amidon waxy d'une matière sèche finale comprise entre 35 et 40 %,
2) hydrolyser par voie acide le lait d'amidon waxy ainsi obtenu et éventuellement, compléter cette hydrolyse acide par une hydrolyse enzymatique à l'aide d'alpha-amylase bactérienne jusqu'à un D.E. compris entre 9 et 14,
3) réaliser sur l'hydrolysat d'amidon ainsi obtenu une étape de traitement au charbon actif et/ou au noir granulaire,
4) mettre en oeuvre une filtration stérilisante consistant en deux filtrations membranaires de diamètre de pore de 0,45 µm puis 0,22 µm,
5) chromatographier sur résines cationiques fortes macroporeuses sous forme alcaline ou alcalino-terreuse cet hydrolysat
6) collecter les polymères de glucose exclus lors de cette étape de chromatographie,
7) mettre en oeuvre sur ces polymères de glucose une étape de traitement thermique à une température de 120°C pendant 2 minutes,
8) réaliser une étape de traitement au charbon actif et/ou au noir granulaire,
9) mettre en oeuvre une filtration stérilisante consistant en deux filtrations membranaires de diamètre de pore de 0,45 µm puis 0,22 µm,
10) réaliser une ultrafiltration d'un seuil de coupure compris entre 30.000 et 100.000 daltons.

## Patentansprüche

1. Verfahren zur Reinigung von Glukosepolymeren, die zur Herstellung von peritonealen Dialyselösungen bestimmt sind, **dadurch gekennzeichnet, dass** es umfasst:
- mindestens einen Behandlungsschritt mit Aktivkohle und/oder mit körnigem Ruß
- mindestens einen Sterilfiltrationsschritt bestehend aus zwei Membranfiltrationen mit einem Porendurchmesser von 0,45 µm und dann 0,22 µm,
- mindestens einen Wärmebehandlungsschritt bestehend aus Erwärmen auf eine Temperatur zwischen 100 und 130 °C für 1 bis 5 Minuten und
- mindestens einen Ultrafiltrationsschritt, wobei die Ultrafiltrationsmembran eine Ausschlussgrenze zwischen 30.000 und 100.000 Dalton aufweist.

2. Verfahren nach Anspruch 1, **dadurch gekennzeichnet, dass** der Wärmebehandlungsschritt aus Erwärmen auf eine Temperatur von 120 °C für 2 Minuten besteht.

3. Verfahren nach einem der Ansprüche 1 bis 2, **dadurch gekennzeichnet, dass** die Behandlung mit Aktivkohle oder mit körnigem Ruß aus zwei Stufen besteht, Erstere bestehend aus Aktivkohle, Zweitere bestehend aus Aktivkohle oder aus körnigem Ruß.

4. Verfahren nach einem der Ansprüche 1 bis 3, **dadurch gekennzeichnet, dass** die Ultrafiltrationsmembran eine Ausschlussgrenze der Ordnung 50.000 Dalton aufweist.

5. Verfahren nach Anspruch 1, **dadurch gekennzeichnet, dass** es besteht aus:
1) Erhalten einer Wachsstärkemilch mit einer endgültigen Trockenmasse zwischen 35 und 40 %,
2) Hydrolysieren der auf diese Weise erhaltenen Wachsstärkemilch mittels Säure und gegebenenfalls vervollständigen dieser sauren Hydrolyse mittels einer enzymatischen Hydrolyse unter Verwendung bakterieller alpha-Amylase bis zu einem DE zwischen 9 und 14,
3) Durchführen eines Behandlungsschritts mit Aktivkohle und/oder körnigem Ruß an dem auf diese Weise erhaltenen Stärkehydrolysat,
4) Durchführen einer Sterilfiltration, bestehend aus zwei Membranfiltrationen mit einem Porendurchmesser von 0,45 µm und dann 0,22 µm,
5) Durchführen einer Chromatographie dieses Hydrolysats an kationischen stark makroporösen Harzen in alkalischer oder erdalkalischer Form;
6) Sammeln der Glukosepolymere, die während dieses Chromatographieschrittes ausgeschlossen wurden,
7) Durchführen eines Wärmebehandlungsschritts an diesen Glukosepolymeren bei einer Temperatur von 120 °C für 2 Minuten,
8) Durchführen eines Behandlungsschritts mit Aktivkohle und/oder körnigem Ruß,
9) Durchführen einer Sterilfiltration, bestehend aus zwei Membranfiltrationen mit einem Porendurchmesser von 0,45 µm und dann 0,22 µm,
10) Durchführen einer Ultrafiltration mit einer Ausschlussgrenze zwischen 30.000 und 100.000 Dalton.

## Claims

1. A method for purifying glucose polymers for the production of peritoneal dialysis solutions, **characterized in that** it comprises :
- at least one step of treatment with activated carbon and/or with granular black carbon,
- at least one sterilizing filtration step consisting of two membrane filtrations where the pore diameter is 0.45µm then 0.22µm,
- at least one heat treatment step consisting in heating at a temperature of between 100 and 130°C for 1 to 5 minutes, and
- at least one ultrafiltration step, the ultrafiltration membrane having a cut-off threshold of between 30.000 and 100.000 daltons.

2. The method as claimed in claim 1, **characterized in that** the heat treatment step consists in heating at a temperature of 120°C for 2 minutes.

3. The method as claimed in either of claims 1 and 2, **characterized in that** the treatment with activated carbon or with granular black carbon consists of two stages, the first composed of activated carbon, the second composed of activated carbon or of granular black carbon.

4. The method as claimed in any one of claims 1 to 3, **characterized in that** the ultrafiltration membrane has a cut-off threshold of between 50.000 daltons.

5. The method as claimed in claim 1, **characterized in that** it consists in:
1) obtaining a waxy starch milk with a final dry matter content of between 35 and 40%,
2) subjecting the resulting waxy starch milk to acid hydrolysis and, optionally, adding to this acid hydrolysis by means of an enzymatic hydrolysis with bacterial alpha-amylase to give a DE between 9 and 14,
3) carrying out on the resulting starch hydrolyzate a step of treatment with activated carbon and/or with granular black carbon,
4) implementing a sterilizing filtration consisting of two membrane filtrations where the pore diameter is 0,45pm then 0,22pm,
5) chromatographing this hydrolyzate on macroporous strong cationic resins in alkali metal or alkaline-earth metal form,
6) collecting the glucose polymers excluded during this chromatography step,
7) implementing on these glucose polymers a step of heat treatment at a temperature of 120°C for 2 minutes,
8) carrying out a step of treatment with activated carbon and/or with granular black carbon,
9) implementing a sterilizing filtration consisting of two membrane filtrations where the pore diameter is 0,45µm then 0,22µm,
10) carrying out an ultrafiltration with a cut-off threshold of between 30.000 and 100.000 daltons.
